# EUROPEAN PATENT SPECIFICATION

(11) **EP 3 329 831 B1**
(45) Date of publication and mention of the grant of the patent: **06.05.2020**
(21) Application number: 17159811.3
(22) Date of filing: 08.03.2017
(51) Int. Cl.: A61B 1/005, A61B 1/04, A61B 1/06, A61B 1/307, A61B 1/00

(54) **URETEROSCOPE FOR DUSTING STONES IN A BODY CAVITY**
URETHROSKOP ZUM ZERSTÄUBEN VON STEINEN IN EINER KÖRPERHÖHLE
URETÈROSCOPE DE POUDRAGE DE PIERRES DANS UNE CAVITÉ CORPORELLE

(30) Priority: 30.11.2016 IN 201641041001
(43) Date of publication of application: 06.06.2018
(73) Proprietor: Wipro Limited, 560 035 Karnataka (IN)
(72) Inventor: MISRA, Pavan, 560094 Bangalore (IN); SELVAM, Gunalan, 560035 Bangalore (IN); BABU, Brijesh, 560016 Bangalore (IN); GAVADE, Sandesh, 560037 Bangalore (IN)
(74) Representative: Finnegan Europe LLP

(56) References cited:
- CN-A- 103 405 261
- US-A1- 2011 015 483
- US-A1- 2015 112 144
- US-A1- 2015 327 750
- US-A1- 2016 038 002

## Description

### FIELD OF THE DISCLOSURE

The present subject matter generally relates to the field of ureteroscopes. More particularly, the present disclosure discloses a ureteroscope and a method for dusting stones in a body cavity.

### BACKGROUND

Ureteroscopy is a method used in treatment of ureteral stones and kidney stones using ureteroscopes. The ureteroscopes have reduced in size and become technologically advanced to facilitate ureteroscopic management of the ureteral stones and the kidney stones.

Currently, a urologist is required to manually maneuver a deflecting tip of a ureteroscope when inserting the ureteroscope into a natural path of a patient to a target stone either in a ureter or a kidney. The urologist may perform a method of physically twitching and tweaking a laser fiber to break the stone. This method is also referred to as dusting. Dusting refers to a process in which the deflecting tip having the laser fiber is swayed from one side to another and a low energy high frequency laser pulse is used to break down the stone into a fine dust, without breaking the stone into big fragments. The fine dust is washed away, by saline flowing in through the ureteroscope, out of a urinary system of the patient through the natural path. Such a method of dusting can be a tedious task and also adds to a load on the urologist and the patient due to the long duration of the dusting process. Such a procedure of dusting also has an increased probability of damaging internal tissues of the urinary system if performed incorrectly or due to poor control of the deflection of the deflecting tip. Moreover, longer procedure time can also affect post-op recovery times and the clinical outcome of the procedure.

With the urologist manually managing the laser fiber by physically twitching and tweaking the laser fiber, there are chances of breakage of the laser fiber which is brittle. It is also difficult for the urologist to maneuver the laser fiber and ensure that laser energy from the laser fiber is directed at the stone without damaging surrounding tissue. If the laser fiber breaks, the urologist has to remove the laser fiber from the ureteroscope and insert another laser fiber, which may happen repeatedly. Such repeated replacement of the laser fiber is costly, as laser fibers are expensive. Further, deflection of the deflecting tip using pull wires by only human effort requires application of significant physical forces on a deflection lever. Such physical effort disrupts the concentration of the urologist and might cause more stress.

Document US 2011/0015483 discloses an endoscopic robotic catheter system for use in kidney stone interventions. Document US 2015/0327750 discloses an endoscopic device and method of controlling such a device. The endoscopic device comprises an imaging unit and gripping jaws. The jaws can be opened and closed using a control system comprising levers, motors and operating wires. A manipulator comprises joints, joint motors and angle detection sensors.

### SUMMARY

The invention is defined by the ureteroscope of independent claim 1. Preferred embodiments are defined by the dependent claims. Any disclosed methods are merely exemplary and do not fall within the scope of the present invention. Any example or embodiment which does not fall under the scope of the claims is not part of the invention. Embodiments of present disclosure disclose a ureteroscope for dusting stones in a body cavity. The ureteroscope includes a flexible probe and a handle. The handle extends proximally from the flexible probe. The flexible probe includes a proximal end and a distal end. The flexible probe includes a working channel and a deflecting tip. The working channel is configured to accommodate a laser fiber in the distal end of the flexible probe at a working position. The deflecting tip is at the distal end of the flexible probe and is configured to be deflected and in turn deflect the laser fiber. The deflecting tip is further positioned at a minimum distance from the target stone. The handle includes a plurality of selection switches and a deflection control module. The plurality of selection switches is configured to provide digital dusting signals. The digital dusting signals correspond to selection of a dusting amplitude and a dusting frequency of the deflecting tip by a user. The deflection control module is configured to control a motor assisted movement of the deflecting tip for the dusting in response to the digital dusting signals. The motor assisted movement is associated with a deflection direction and a deflection magnitude of the deflecting tip.

Disclosed herein is a method of dusting stones in a body cavity by a ureteroscope. The method includes receiving, by the processing module, digital dusting signals from a plurality of selection switches, the digital dusting signals corresponding to a dusting amplitude and a dusting frequency of a deflecting tip at a distal end of a flexible probe of the ureteroscope, the digital dusting signals being received subsequent to deflection of the deflecting tip to position the deflecting tip at a minimum distance from a target stone. The method also includes processing, by the processing module, the digital dusting signals to generate an analog dusting signal. Further, the method includes initiating, by the processing module, activation of a motor in the deflection control module in response to the analog dusting signal. The analog dusting signal is used to deflect the deflecting tip using a plurality of control wires and in turn to deflect a laser fiber extending from the deflecting tip to enable dusting of the target stone.

The method may be performed using a ureteroscope having a deflection control module that is configured to control a motor assisted movement of the deflecting tip for the dusting in response to the digital dusting signals, the motor assisted movement being associated with the deflection direction and the deflection magnitude of the deflecting tip. The method may further comprising determining, by the processing module, a type of the dusting of the target stone, the type of dusting comprising one of a fine dusting of the target stone and a coarse dusting of the target stone.

The present disclosure also includes a processor-readable medium comprising instructions that, when executed by a processing module of a ureteroscope, cause the processing module to perform a method as disclosed herein.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

The accompanying drawings, which are incorporated in and constitute a part of this disclosure, illustrate exemplary embodiments and, together with the description, serve to explain the disclosed principles. In the figures, the left-most digit(s) of a reference number identifies the figure in which the reference number first appears. The same numbers are used throughout the figures to reference like features and components. Some embodiments of system and/or methods in accordance with embodiments of the present subject matter are now described, by way of example only, and with reference to the accompanying figures, in which:
FIG. 1 illustrates a side view of a ureteroscope for dusting stones in a body cavity, in accordance with some embodiments of the present disclosure;
FIG. 2A illustrates a deflecting tip of a ureteroscope, in accordance with some embodiments of the present disclosure;
FIG. 2B illustrates a cross-sectional view of a deflecting tip of a ureteroscope, in accordance with some embodiments of the present disclosure;
FIG. 3 illustrates a side view of a ureteroscope for dusting stones in a body cavity, in accordance with other embodiments of the present disclosure;
FIG. 4 illustrates a block diagram of a processing module in a ureteroscope, in accordance with some embodiments of the present disclosure; and
FIG. 5 is a flow diagram illustrating a method of dusting stones in a body cavity, in accordance with some embodiments of the present disclosure.

It should be appreciated by those skilled in the art that any block diagrams herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

### DETAILED DESCRIPTION

In the present document, the word "exemplary" is used herein to mean "serving as an example, instance, or illustration." Any embodiment or implementation of the present subject matter described herein as "exemplary" is not necessarily to be construed as preferred or advantageous over other embodiments.

While the disclosure is susceptible to various modifications and alternative forms, specific embodiment thereof has been shown by way of example in the drawings and will be described in detail below. It should be understood, however that it is not intended to limit the disclosure to the particular forms disclosed, but on the contrary, the disclosure is to cover all modifications, equivalents, and alternative falling within the scope of the disclosure.

The terms "comprises", "comprising", or any other variations thereof, are intended to cover a non-exclusive inclusion, such that a setup, device or method that comprises a list of components or steps does not include only those components or steps but may include other components or steps not expressly listed or inherent to such setup or device or method. In other words, one or more elements in a system or apparatus proceeded by "comprises... a" does not, without more constraints, preclude the existence of other elements or additional elements in the system or apparatus.

In the following detailed description of the embodiments of the disclosure, reference is made to the accompanying drawings that form a part hereof, and in which are shown by way of illustration specific embodiments in which the disclosure may be practiced. These embodiments are described in sufficient detail to enable those skilled in the art to practice the disclosure, and it is to be understood that other embodiments may be utilized and that changes may be made without departing from the scope of the present disclosure. The following description is, therefore, not to be taken in a limiting sense.

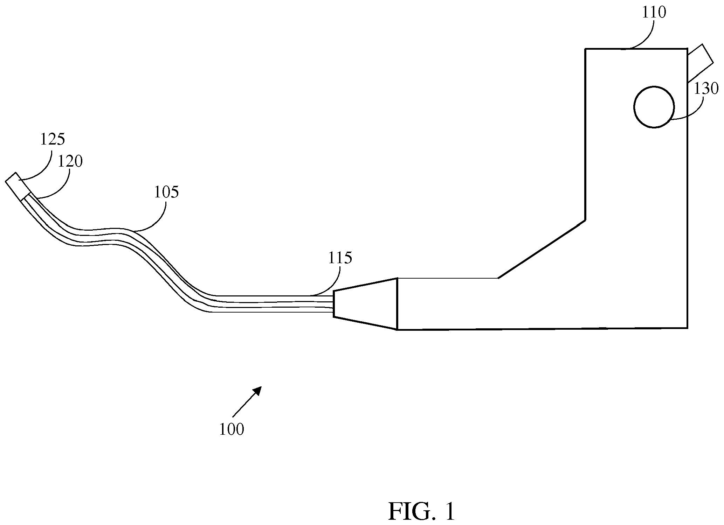

FIG. 1 illustrates a side view of a ureteroscope 100 for dusting stones in a body cavity, in accordance with some embodiments of the present disclosure.

The ureteroscope 100 includes a flexible probe 105 and a handle 110. The handle 110 extends proximally from the flexible probe 105. The flexible probe 105 includes a proximal end 115 and a distal end 120. The proximal end 115 of the flexible probe 105 is coupled to the handle 110. The flexible probe 105 includes at least one working channel (not shown in FIG. 1) and a deflecting tip 125. The deflecting tip 125 of the flexible probe 105 is coupled to the distal end 120. The flexible probe 105 further includes an image viewing system (not shown in FIG. 1) and a light source (not shown in FIG. 1). The handle 110 includes a plurality of selection switches, for example a selection switch 130, on an exterior portion of the handle 110. The handle 110 further includes a deflection control module (not shown in FIG. 1) in an interior portion of the handle 110.

The ureteroscope 100 can be used in a stone removal operation from a kidney of a patient. A user, for example a doctor or other medical personnel, inserts the flexible probe 105 of the ureteroscope 100 into a urethra of the patient. The user advances the flexible probe 105 such that the deflecting tip 125 passes into and through a urinary bladder, into and through a ureter, and into a kidney of the patient.

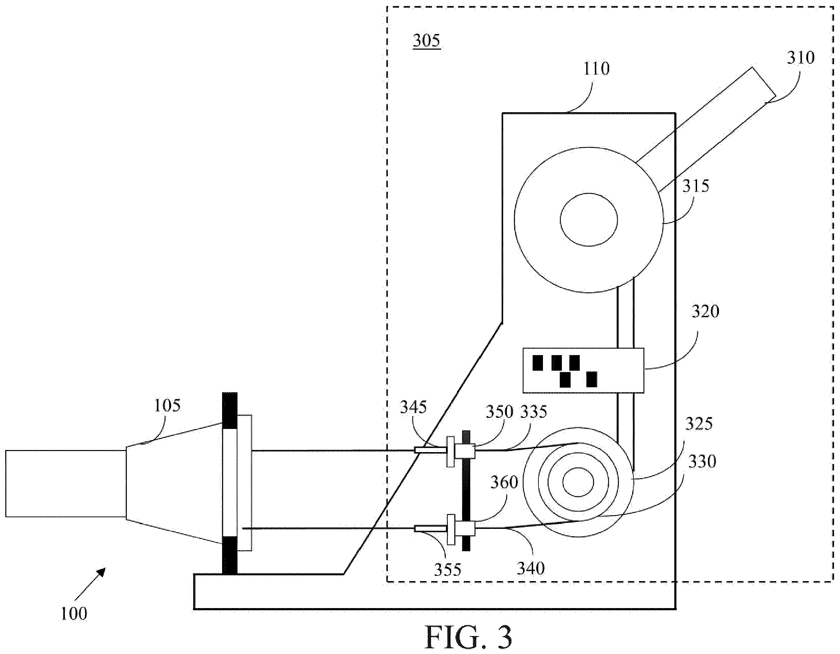

The user positions the deflecting tip 125 of the ureteroscope 100 within the kidney at a minimum distance from a target stone. The target stone is found by deflection of the deflecting tip 125 and is described in detail with reference to FIG. 3. In some embodiments, the angle of deflection of the deflecting tip 125 may range between ±270 degrees, allowing for visualization of an upper urinary tract including each renal calyx.

Once the deflecting tip 125 of the ureteroscope 100 is positioned in the kidney, a laser fiber is received into a working channel of the flexible probe 105 and accommodated in the distal end 120 of the flexible probe 105 at an initial position. In an example the laser fiber may include a diameter of 200 micrometer, 272 micrometer, 365 micrometer, 550 micrometer, or 1000 micrometer. The laser fiber is further positioned at a working position. Herein, the 'working position' is referred to as a predefined distance of the laser fiber from the distal end 120 of the flexible probe 105 defined by laser energy settings and stone composition. The user subsequently operates the selection switch 130 to control the deflecting tip 125 and thereby control movement of the laser fiber for dusting the target stone in the kidney. The deflecting tip 125 is moved in a similar repetitive motion for dusting the target stone.

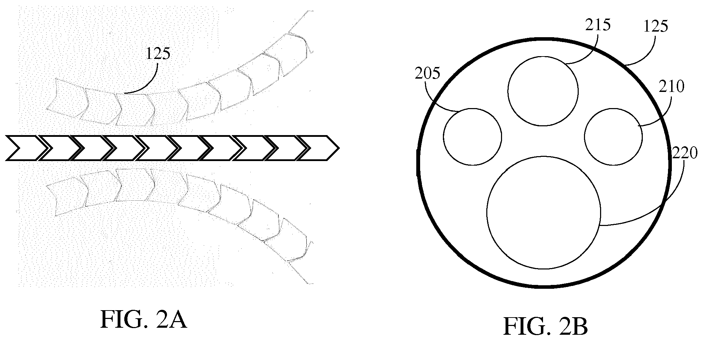

In some embodiments, the ureteroscope 100 may also be positioned in a ureter of the patient for dusting the target stone in the ureter. The deflecting tip 125 is explained in detail with reference to FIG. 2A and FIG. 2B.

Referring now to FIG. 2A, the deflecting tip 125 is illustrated, in accordance with some embodiments of the present disclosure. In the illustrated FIG. 2A, the deflecting tip 125 is shown to be composed of a plurality of segmented portions, for example 25 segmented portions. Each segmented portion includes a couple of through holes to pass a corresponding control wire. Each segmented portion is coupled to another segmented portion sequentially using the plurality of control wires such that the deflecting tip 125 is flexible.

Referring now to FIG. 2B, a cross sectional view of the deflecting tip 125 is illustrated, in accordance with some embodiments of the present disclosure. As illustrated in FIG. 2B, the deflecting tip 125 includes a working channel 205 and a working channel 210. The working channel 205 is configured to accommodate the laser fiber for dusting the target stone. In some embodiments, the working channel 205 may accommodate a 3 french gauge (Fr) size working device. In other embodiments, the working channel 205 may be used to accommodate baskets, grasping forceps, and the like. The working channel 210 is configured to be used for irrigation, for example with saline, to wash away dust after the dusting of the target stone.

The deflecting tip 125 further includes an image viewing system 215 and a light source 220. In one example, the image viewing system 215 is a complementary metal oxide semiconductor (CMOS) based image viewing system. The image viewing system 215 is configured to view the laser fiber extending from the deflecting tip 125 and also the target stone. The light source 220 is configured to provide light to assist viewing of the target stone and the laser fiber through the image viewing system 215.

The deflection of the deflecting tip 125 of the ureteroscope 100 and subsequent dusting using the laser fiber is explained in detail with reference to FIG. 3.

FIG. 3 illustrates a side view of the ureteroscope 100 for dusting stones in a body cavity of a patient, in accordance with other embodiments of the present disclosure. In the illustrated FIG. 3, a deflection control module 305 of the handle 110, the deflection of the deflecting tip 125 and the dusting of the target stone are described herein in detail. The deflection control module 305 is configured to control a motor assisted movement of the deflecting tip 125 for the dusting. The motor assisted movement is associated with a deflection direction and a deflection magnitude of the deflecting tip 125.

The handle 110 includes the deflection control module 305 (an electromechanical module) in the interior portion of the handle 110. The deflection control module 305 includes a deflection lever 310, a potentiometer 315, a processing module 320, a motor 325, a deflection pulley 330, and a plurality of control wires, for example a first control wire 335 and a second control wire 340.

The potentiometer 315 is coupled to the deflection lever 310. In some embodiments, the potentiometer 315 is used to overcome usage of increased physical force for deflecting or dusting the deflecting tip 125. The processing module 320 is coupled to the potentiometer 315. The motor 325, for example a stepper motor, is coupled to the processing module 320. In an example, the motor 325 may be associated with a step angle of 1.8 degrees, a holding torque of 21 Newton-centimeters, and a rotor inertia of 0.035 kilogramme square centimeter. The deflection pulley 330 is coupled to the motor 325. The plurality of control wires, for example the first control wire 335 and the second control wire 340, is coupled between the deflection pulley 330 and the deflecting tip 125 of the flexible probe 105 (that is segmented, as illustrated in FIG. 2A, to allow bending). In an example the first control wire 335 and the second control wire 340 are nitinol (a metal alloy of nickel and titanium) wires. The first control wire 335 and the second control wire 340 are attached to the deflection pulley 330 such that when the first control wire 335 is pulled, the second control wire 340 is slackened and when the second control wire 340 is pulled, the first control wire 335 is slackened.

In some embodiments, the first control wire 335 is routed through a first sleeve 345. The first sleeve 345 is housed within a first static wall support 350 to allow the first control wire 335 to move without friction against the flexible probe 105. Similarly, the second control wire 340 is routed through a second sleeve 355. The second sleeve 355 is housed within a second static wall support 360 to allow the second control wire 325 to move without friction against the flexible probe 105.

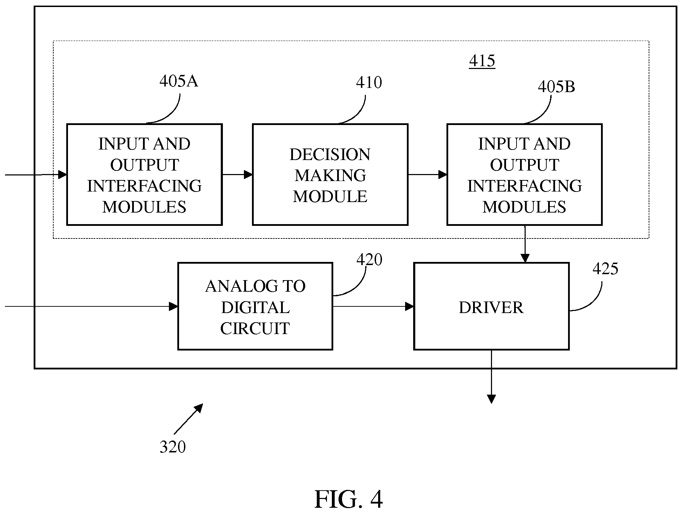

The flexible probe 105 of the ureteroscope 100 is inserted into the body cavity of the patient and the defecting tip 125 is deflected or maneuvered to reach a position of the target stone in the kidney or the ureter of the patient. The deflection of the deflecting tip 125 is performed using the deflection control module 305. The deflection lever 310 is first deflected by the user to deflect the deflecting tip 125. The potentiometer 315 turns a number of turns in response to deflection of the deflection lever 310. The number of turns define a variable resistance, for example of 1 kilo ohm, associated with deflecting the deflecting tip 125. The processing module 320 senses the number of turns of the potentiometer 315 to generate a digital deflection signal. The processing module 320 further converts the digital deflection signal to generate an analog deflection signal. The analog deflection signal drives the motor 325. Functions of the processing module 320 during the deflection of the deflecting tip 125 is explained in detail with reference to FIG. 4.

The motor 325 is configured to turn a number of steps based on the analog deflection signal. The deflection pulley 330 coupled, for example, on a shaft of the motor 325, operates in accordance with the number of steps turned by the motor and hence controls the deflection of the deflecting tip 125 using the first control wire 335 and the second control wire 340. The first control wire 335 and the second control wire 340 attached to the deflecting tip 125 (as explained with reference to FIG. 2A) allow deflection of the deflecting tip 125 and thereby the laser fiber.

In some embodiments, if the user requires to bend the deflecting tip 125 left, the user deflects the deflection lever 310 to a left position. The first control wire 335 is tensed in response to the deflection lever 310 being deflected to the left position and the second control wire 340 becomes slackened to enable the deflecting tip 125 to bend left in accordance to an amount of turn in the deflection lever 310.

Similarly, in some embodiments, if the user requires to bend the deflecting tip 125 right, the user deflects the deflection lever 310 to a right position. The second control wire 340 is tensed in response to the deflection lever 310 being deflected to the right position and the first control wire 335 becomes slackened to enable the deflecting tip 125 to bend right in accordance to the amount of turn in the deflection lever 310.

Subsequent to the deflection of the deflecting tip 125 to position the deflecting tip 125 at the minimum distance from the target stone, the laser fiber is energized to a power level and a frequency level to enable the dusting of the target stone. The dusting of the target stone in the body cavity of the patient is explained in detail herein. The user, for example the doctor or other medical personnel, selects a dusting amplitude and a dusting frequency of the deflecting tip 125, as required, by operating the plurality of selection switches, for example the selection switch 130. The dusting amplitude and the dusting frequency selected by the user corresponds to digital dusting signals provided by the plurality of selection switches to the processing module 320.

The processing module 320 processes the digital dusting signals to generate an analog dusting signal. The processing module 320 further initiates activation of the motor 325 in the deflection control module 305 in response to the analog dusting signal. The functions of the processing module 320 during the dusting is explained in detail with reference to FIG. 4. The deflecting tip 125 is hence deflected using the first control wire 335 and the second control wire 340 and in turn the laser fiber extending from the deflecting tip 125 in turn gets deflected to enable the dusting of the target stone.

In some embodiments, a laser trigger is used to energize the laser fiber and is a pedal activated module which is located away from the ureteroscope 100. In other embodiments, the laser trigger may be included within the ureteroscope 100.

In some embodiments, the image viewing system 215 and the light source 220 provides aid to the user to position the deflecting tip 125 near the target stone and to perform the dusting of the target stone.

Referring now to FIG. 4, the processing module 320 is illustrated, in accordance with some embodiments of the present disclosure. The processing module 320 includes a plurality of input and output interfacing modules, for example input and output interfacing modules 405A and input and output interfacing modules 405B. The processing module 320 further includes a decision making module 410. In addition, the processing module 320 includes a controller 415. The controller 415 includes an ADC 420 and a driver 425. In an example, the ADC 420 has a range of values from 0 to 1023.

The decision making module 410 is coupled between the input and output interfacing modules 405A and the input and output interfacing modules 405B. The driver 425 is coupled to the input and output interfacing modules 405B and the ADC 420. An output of the driver 425 is further coupled to the motor 325.

During the deflection of the deflecting tip 125, the ADC 420 in the processing module 320 senses the number of turns of the potentiometer 315 to generate the digital deflection signal. The digital deflection signal defines the deflection direction and the deflection magnitude. The driver 425 receives the digital deflection signal and in turn generates the analog deflection signal.

The motor 325, coupled to the driver 425, hence turns the number of steps in accordance with the analog deflection signal. The deflecting tip 125 is thereby deflected by controlling the plurality of control wires using the deflection pulley 330 coupled to the motor 325. In some embodiments, an amount of deflection of the deflecting tip 125 depends on a number of segmented portions in the deflecting tip 125 and architecture or construction of the deflecting tip 125.

During the dusting of the target stone, the input and output interfacing modules 405A receive the digital dusting signals from the plurality of selection switches selected by the user. The digital dusting signals are forwarded to the decision making module 410. The decision making module 410 determines a type of the dusting, for example a fine dusting or a coarse dusting, required for the target stone based on the digital dusting signals. The decision making module 410 further generates a digital dusting signal.

The digital dusting signal obtained from the decision making module 410 is fed to the driver 425 by the input and output interfacing modules 405B. The driver 425 receives the digital dusting signal and in turn generates an analog dusting signal. The motor 325, coupled to the driver 425, hence turns the number of steps in accordance with the analog dusting signal. The deflecting tip 125 is deflected by controlling the plurality of control wires using the deflection pulley 330 coupled to the motor 325. The laser fiber is thereby deflected in a continuous swaying motion (for example, by programming the controller 415 to a sinusoidal signal) and if the user is satisfied with the deflection, the laser fiber is energized and the dusting of the target stone is performed.

In some embodiments, the deflection direction and the deflection magnitude of the deflection is effected by programming the controller 415 to a sinusoidal signal.

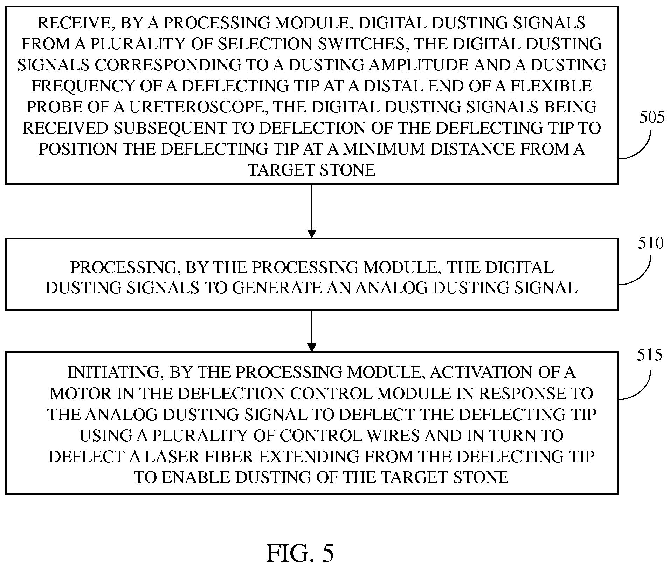

FIG. 5 is a flow diagram illustrating a method 500 of dusting stones in a body cavity by a ureteroscope, for example the ureteroscope 100 of FIG. 1, in accordance with some embodiments of the present disclosure. The term 'dusting' refers to a technique for disintegrating stones in a kidney or a ureter of a patient into fine fragments with a laser fiber by moving a deflecting tip of the ureteroscope, for example the deflecting tip 125 of the ureteroscope 100 of FIG. 1.

The order in which the method is described is not intended to be construed as a limitation, and any number of the described method blocks can be combined in any order to implement the method. Additionally, individual blocks may be deleted from the method without departing from the scope of the subject matter described herein.

At step 505, the method 500 includes receiving, by a processing module, for example the processing module 320 of FIG. 3, digital dusting signals from a plurality of selection switches, for example a selection switch 130 of FIG. 1, selected by a user. The digital dusting signals correspond to a dusting amplitude and a dusting frequency of a deflecting tip, for example a deflecting tip 125 of FIG. 1, at a distal end of a flexible probe of the ureteroscope. The digital dusting signals are received subsequent to deflection of the deflecting tip to position the deflecting tip at a minimum distance from a target stone. The ureteroscope is positioned in the proper position for dusting the target stone when the deflecting tip is at the minimum distance from the target stone.

In some embodiments, the method of the deflection of the deflecting tip is explained herein. The method includes generating, by the processing module, a digital deflection signal based on deflection of a deflection lever, for example the deflection lever 305 of FIG. 3. The digital deflection signal defines a deflection direction and a deflection magnitude of the deflecting tip.

In some embodiments, the digital deflection signal is generated by determining a number of turns of a potentiometer, for example the potentiometer 310 of FIG. 3, in response to deflection of the deflection lever. The number of turns further define a variable resistance associated with deflecting the deflecting tip. Further, the digital deflection signal is generated, by the processing module, based on the number of turns of the potentiometer.

The method also includes processing, by the processing module, the digital deflection signal to generate an analog deflection signal.

The method further includes initiating, by the processing module, driving of the motor, for example the motor 325 of FIG. 3, to turn a number of steps based on the analog deflection signal. In an example, the motor used is a stepper motor. The driving of the motor deflects the deflecting tip using the plurality of control wires, for example the first control wire 335 and the second control wire 340 of FIG. 3, and a deflection pulley, for example the deflection pulley 330 of FIG. 3, to position the deflecting tip at the minimum distance from the target stone.

At step 510, the method 500 includes processing, by the processing module, the digital dusting signals to generate an analog dusting signal. The method of generating the analog dusting signal is explained with reference to FIG. 4 is not explained herein for sake of brevity.

At step 515, the method 500 includes initiating, by the processing module, activation of the motor in the deflection control module in response to the analog dusting signal. The deflecting tip is deflected using the plurality of control wires and in turn the laser fiber extending from the deflecting tip is deflected to enable the dusting of the target stone. The laser fiber is energized to a power level and a frequency level to enable the dusting of the target stone.

In some embodiments, the laser fiber is energized using a laser trigger that is a pedal activated module and which is located away from the ureteroscope. In other embodiments, the laser fiber is energized using the laser trigger included within the ureteroscope.

In some embodiments, a type of dusting of the target stone is determined by the processing module. In an example the type of dusting includes one of a fine dusting of the target stone or a coarse dusting of the target stone.

In some embodiments, various dusting amplitudes and dusting frequencies may be pre-programmed and may be selected using a selection switch on the ureteroscope.

In some embodiments, after the dusting of the target stone, dust of the target stone is flushed outside the body using the ureteroscope without affecting adjacent body tissues.

Embodiments of the present disclosure provide a ureteroscope and a method for dusting stones in a body cavity. The present disclosure provides a controlled and motorized sinusoidal sweeping of a deflecting tip of the ureteroscope for the dusting of a target stone in a kidney or a ureter. Such controlled deflection and dusting has finer and precise movements by using a motor as compared to random physical twitching of the laser fiber in existing procedures. The present disclosure also allows flexibility of accommodating automation components in current form factor without much increase in size and weight of the ureteroscope, thereby being customizable as per need.

The ureteroscope in the present disclosure is a generic device that may be applied in different flexible ureteroscopes which are used in places where flexible ureteroscopy is performed. The present disclosure of providing an automated movement of the deflecting tip may also be applied to various flexible scopes which are used for different minimally invasive surgeries, for example a laparoscopic procedure.

The terms "an embodiment", "embodiment", "embodiments", "the embodiment", "the embodiments", "one or more embodiments", "some embodiments", and "one embodiment" mean "one or more (but not all) embodiments of the invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

A description of an embodiment with several components in communication with each other does not imply that all such components are required. On the contrary a variety of optional components are described to illustrate the wide variety of possible embodiments of the invention.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features. Thus, other embodiments of the invention need not include the device itself.

The illustrated operations of FIG. 5 show certain events occurring in a certain order. In alternative embodiments, certain operations may be performed in a different order, modified or removed. Moreover, steps may be added to the above described logic and still conform to the described embodiments. Further, operations described herein may occur sequentially or certain operations may be processed in parallel. Yet further, operations may be performed by a single processing unit or by distributed processing units.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based here on. Accordingly, the disclosure of the embodiments of the invention is intended to be illustrative, but not limiting, of the scope of the invention, which is set forth in the following claims.

While various aspects and embodiments have been disclosed herein, other aspects and embodiments will be apparent to those skilled in the art. The various aspects and embodiments disclosed herein are for purposes of illustration and are not intended to be limiting, with the scope being defined by the following claims.

## Claims

1. A ureteroscope for dusting stones in a body cavity, the ureteroscope comprising:
a flexible probe comprising a proximal end and a distal end, the flexible probe comprising:
a working channel configured to accommodate a laser fiber in the distal end of the flexible probe at a working position, the laser fiber to be deflected against a target stone for dusting; and
a deflecting tip at the distal end of the flexible probe and configured to be deflected and in turn deflect the laser fiber, thereby allowing the deflecting tip to be positioned at a minimum distance from the target stone; and
a handle extending proximally from the flexible probe, the handle comprising:
a plurality of selection switches configured to provide digital dusting signals, the digital dusting signals corresponding to selection of a dusting amplitude and a dusting frequency of the deflecting tip by a user; and
a deflection control module configured to control a motor assisted movement of the deflecting tip for the dusting in response to the digital dusting signals, the motor assisted movement associated with a deflection direction and a deflection magnitude of the deflecting tip; wherein
the deflection control module comprises:
a deflection lever configured to be deflected by the user to deflect the deflecting tip; **characterised in that** the deflection control module further comprises a potentiometer coupled to the deflection lever and configured to turn a number of turns in response to deflection of the deflection lever, the number of turns further defining a variable resistance associated with deflecting the deflecting tip;
a processing module coupled to the potentiometer and comprising:
an analog to digital converter (ADC) configured to sense the number of turns of the potentiometer to generate a digital deflection signal, the digital deflection signal defining the deflection direction and the deflection magnitude; and
a driver coupled to the ADC and configured to generate an analog deflection signal in response to the digital deflection signal;
the deflection control module further comprising:
a motor coupled to the processing module and configured to turn a number of steps based on the analog deflection signal;
a deflection pulley coupled to the motor and configured to control the deflection of the deflecting tip; and
a plurality of control wires coupled between the deflection pulley and the deflecting tip of the flexible probe, the plurality of control wires comprising a first control wire and a second control wire, the first control wire and the second control wire configured to allow the deflecting of the deflecting tip and in turn the laser fiber based on movement of the deflection pulley.

2. The ureteroscope as claimed in claim 1, wherein the flexible probe further comprises:
an image viewing system configured to view the laser fiber extending from the deflecting tip and the target stone; and
a light source configured to provide light to assist viewing the laser fiber and the target stone through the image viewing system.

3. The ureteroscope as claimed in claim 1, wherein the processing module further comprises:
a controller comprising:
a plurality of input and output interfacing modules configured to obtain the digital dusting signals from the plurality of selection switches; and
a decision making module configured to determine a type of the dusting of the target stone, the type of dusting comprising one of a fine dusting of the target stone and a coarse dusting of the target stone.

4. The ureteroscope as claimed in claim 1 or 3, wherein the deflection pulley coupled to the motor, is configured to deflect the deflecting tip by controlling the plurality of control wires in a continuous swaying motion, thereby deflecting the laser fiber in the continuous swaying motion.

5. The ureteroscope as claimed in claim 1 or claim 3, wherein the first control wire is routed through a first sleeve, the first sleeve being housed within a first static wall support to allow the first control wire to move without friction.

6. The ureteroscope as claimed in claim 5, wherein the second control wire is routed through a second sleeve, the second sleeve being housed within a second static wall support to allow the second control wire to move without friction.

7. The ureteroscope as claimed in claim 6, wherein the first control wire is configured to be tensed in response to the deflection lever being deflected to a left position, and the second control wire is configured to be slackened to enable the deflecting tip to bend left in accordance to an amount of turn in the deflection lever.

8. The ureteroscope as claimed in claim 7, wherein the second control wire is configured to be tensed in response to the deflection lever being deflected to a right position, and the first control wire is configured to be slackened to enable the deflecting tip to bend right in accordance to the amount of turn in the deflection lever.

9. The ureteroscope as claimed in any of the preceding claims, wherein the working position is a predefined distance of the laser fiber from the distal end of the flexible probe and is defined by laser energy settings and composition of the target stone.

10. The ureteroscope as claimed in any of the preceding claims, wherein the laser fiber is configured to be energized to a power level and a frequency level to enable the dusting of the target stone.

11. The ureteroscope as claimed in claim 3 or any claim dependent thereon, wherein the deflection direction and the deflection magnitude is effected by programming the controller to a sinusoidal signal.

## Patentansprüche

1. Urethroskop zum Zerstäuben von Steinen in einer Körperhöhle, wobei das Urethroskop umfasst:
eine flexible Sonde, umfassend ein proximales Ende und ein distales Ende, wobei die flexible Sonde umfasst:
einen Arbeitskanal, der konfiguriert ist, um eine Laserfaser im distalen Ende der flexiblen Sonde an einer Arbeitsposition aufzunehmen, wobei die Laserfaser zum Zerstäuben an einem Zielstein gebeugt wird; und
eine Beugungsspitze am distalen Ende der flexiblen Sonde und die konfiguriert ist, um gebeugt zu werden und ihrerseits die Laserfaser zu beugen, wodurch ermöglicht wird, dass die Beugungsspitze in einen Mindestabstand vom Zielstein positioniert wird; und
einen Griff, der sich proximal von der flexiblen Sonde erstreckt, wobei der Griff umfasst:
eine Vielzahl von Auswahlschaltern, die konfiguriert sind, um digitale Zerstäubungssignale bereitzustellen, wobei die digitalen Zerstäubungssignale einer Auswahl einer Zerstäubungsamplitude und einer Zerstäubungsfrequenz der Beugungsspitze durch einen Nutzer entsprechen; und
ein Beugungssteuermodul, das konfiguriert ist, um in Reaktion auf die digitalen Beugungssignale eine motorunterstützte Bewegung der Beugungsspitze zum Zerstäuben zu steuern, wobei die motorunterstützte Bewegung einer Beugungsrichtung und einer Beugungsgröße der Beugungsspitze zugeordnet ist; wobei
das Beugungssteuermodul umfasst:
einen Beugungshebel, der konfiguriert ist, um vom Nutzer gebeugt zu werden, um die Beugungsspitze zu beugen;
**dadurch gekennzeichnet, dass** das Beugungssteuermodul ferner ein Potentiometer umfasst, das mit dem Beugungshebel gekoppelt und konfiguriert ist, um sich in Reaktion auf eine Beugung des Beugungshebels eine Anzahl von Umdrehungen zu drehen, wobei die Anzahl von Umdrehungen ferner einen variablen Widerstand definiert, der dem Beugen der Beugungsspitze zugeordnet ist;
ein Verarbeitungsmodul, das mit dem Potentiometer gekoppelt ist und umfasst:
einen Analog-Digital-Umsetzer (ADC), der konfiguriert ist, um die Anzahl von Umdrehungen des Potentiometers zu erfassen, um ein digitales Beugungssignal zu generieren, wobei das digitale Beugungssignal die Beugungsrichtung und die Beugungsmenge definiert; und
einen Treiber, der mit dem ADC gekoppelt und konfiguriert ist, um in Reaktion auf das digitale Beugungssignal ein analoges Beugungssignal zu erzeugen;
wobei das Beugungssteuermodul ferner umfasst:
einen Motor, der mit dem Verarbeitungsmodul gekoppelt und konfiguriert ist, um sich basierend auf dem analogen Beugungssignal eine Anzahl von Schritten zu drehen;
eine Beugungsrolle, die mit dem Motor gekoppelt und konfiguriert ist, um die Beugung der Beugungsspitze zu steuern; und
eine Vielzahl von Steuerdrähten, die zwischen der Beugungsrolle und der Beugungsspitze der flexiblen Sonde gekoppelt sind, wobei die Vielzahl von Steuerdrähten einen erste Steuerdraht und einen zweiten Steuerdraht umfassen, wobei der erste Steuerdraht und der zweite Steuerdraht konfiguriert sind, um das Beugen der Beugungsspitze und dadurch der Laserfaser basierend auf der Bewegung der Beugungsrolle zu ermöglichen.

2. Urethroskop nach Anspruch 1, wobei die flexible Sonde ferner umfasst:
ein Bildbetrachtungssystem, das konfiguriert ist, um die sich von der Beugungsspitze und dem Zielstein erstreckende Laserfaser zu betrachten; und
eine Lichtquelle, die konfiguriert ist, um Licht bereitzustellen, um die Betrachtung der Laserfaser und des Zielsteins durch das Bildbetrachtungssystem zu unterstützen.

3. Urethroskop nach Anspruch 1, wobei das Verarbeitungsmodul ferner umfasst:
eine Steuerung, umfassend:
eine Vielzahl von Eingangs- und Ausgangs-Schnittstellenmodulen, die konfiguriert sind, um digitale Zerstäubungssignale von der Vielzahl von Auswahlschaltern zu erhalten; und
ein Entscheidungsmodul, das konfiguriert ist, um einen Typ des Zerstäubens des Zielsteins zu bestimmen, wobei der Typ des Zerstäubens einer aus einem feinen Zerstäuben des Zielsteins und einem groben Zerstäuben des Zielsteins ist.

4. Urethroskop nach Anspruch 1 oder 3, wobei die mit dem Motor gekoppelte Beugungsrolle konfiguriert ist, um die Beugungsspitze durch Steuern der Vielzahl von Steuerdrähten in einer ununterbrochenen Schwingungsbewegung zu beugen, wodurch die Laserfaser in der ununterbrochenen Schwingungsbewegung gebeugt wird.

5. Urethroskop nach Anspruch 1 oder Anspruch 3, wobei der erste Steuerdraht durch eine erste Hülse geleitet ist, wobei die erste Hülse in einer ersten statischen Wandstütze aufgenommen ist, um dem ersten Steuerdraht zu ermöglichen, sich ohne Reibung zu bewegen.

6. Urethroskop nach Anspruch 5, wobei der zweite Steuerdraht durch eine zweite Hülse geleitet ist, wobei die zweite Hülse in einer zweiten statischen Wandstütze aufgenommen ist, um dem zweiten Steuerdraht zu ermöglichen, sich ohne Reibung zu bewegen.

7. Urethroskop nach Anspruch 6 wobei der erste Steuerdraht konfiguriert ist, um in Reaktion darauf, dass der Beugungshebel in eine linke Position gebeugt wird, gespannt zu werden, und der zweite Steuerdraht konfiguriert ist, um entspannt zu werden, um der Beugungsspitze zu ermöglichen, sich gemäß einer Menge an Umdrehungen im Beugungshebel nach links zu beugen.

8. Urethroskop nach Anspruch 7, wobei der zweite Steuerdraht konfiguriert ist, um in Reaktion darauf, dass der Beugungshebel in eine rechte Position gebeugt wird, gespannt zu werden, und der erste Steuerdraht konfiguriert ist, um entspannt zu werden, um der Beugungsspitze zu ermöglichen, sich gemäß der Menge an Umdrehungen im Beugungshebel nach rechts zu beugen.

9. Urethroskop nach einem der vorangehenden Ansprüche, wobei die Arbeitsposition ein vordefinierter Abstand der Laserfaser vom distalen Ende der flexiblen Sonde ist und durch Laserenergieeinstellungen und eine Zusammensetzung des Zielsteins definiert wird.

10. Urethroskop nach einem der vorangehenden Ansprüche, wobei die Laserfaser konfiguriert ist, um auf ein Leistungsniveau und ein Frequenzniveau energetisiert zu werden, um das Zerstäuben des Zielsteins zu ermöglichen.

11. Urethroskop nach Anspruch 3 oder einem davon abhängigen Anspruch, wobei die Beugungsrichtung und die Beugungsgröße durch Programmieren der Steuerung auf ein sinusoidales Signal bewirkt wird.

## Revendications

1. Urétéroscope de réduction en poudre de calculs dans une cavité corporelle, l'urétéroscope comprenant :
une sonde souple comprenant une extrémité proximale et une extrémité distale, la sonde souple comprenant :
un canal de travail conçu pour accueillir une fibre laser dans l'extrémité distale de la sonde souple au niveau d'une position de travail, la fibre laser étant destinée à être déviée contre une calcul cible en vue du réduction en poudre ; et
une pointe de déflexion au niveau de l'extrémité distale de la sonde souple et conçue pour être déviée et à son tour dévier la fibre laser, ce qui permet le positionnement de la pointe de déflexion à une distance minimale du calcul cible ; et
une poignée s'étendant de manière proximale depuis la sonde souple, la poignée comprenant :
une pluralité de commutateurs de sélection conçus pour délivrer des signaux de réduction en poudre numériques, les signaux de réduction en poudre numériques correspondant à une sélection d'amplitude de réduction en poudre et de fréquence de réduction en poudre de la pointe de déflexion par un utilisateur ; et
un module de commande de déflexion conçu pour commander le déplacement assisté par moteur de la pointe de déflexion en vue de la réduction en poudre en réponse aux signaux de réduction en poudre numériques, le déplacement assisté par moteur étant associé à une direction de déflexion et à une magnitude de déflexion de la pointe de déflexion ;
ledit module de commande de déflexion comprenant :
un levier de déflexion conçu pour être dévié par l'utilisateur pour dévier la pointe de déflexion ;
**caractérisé en ce que** le module de commande de déflexion comprend en outre un potentiomètre couplé au levier de déflexion et conçu pour tourner d'un certain nombre de tours en réponse à la déflexion du levier de déflexion, le nombre de tours définissant en outre une résistance variable associée à la déflexion de la pointe de déflexion ;
un module de traitement couplé au potentiomètre et comprenant :
un convertisseur analogique/numérique (ADC) conçu pour détecter le nombre de tours du potentiomètre pour générer un signal de déflexion numérique, le signal de déflexion numérique définissant la direction de déflexion et la magnitude de déflexion ; et
un pilote couplé à l'ADC et conçu pour générer un signal de déflexion analogique en réponse au signal de déflexion numérique ;
le module de commande de déflexion comprenant en outre :
un moteur couplé au module de traitement et conçu pour tourner d'un certain nombre de pas en fonction du signal de déflexion analogique ;
une poulie de déflexion couplée au moteur et conçue pour commander la déflexion de la pointe de déflexion ; et
une pluralité de câbles de commande couplés entre la poulie de déflexion et la pointe de déflexion de la sonde souple, la pluralité de câbles de commande comprenant un premier câble de commande et un second câble de commande, le premier câble de commande et le second câble de commande étant conçus pour permettre la déflexion de la pointe de déflexion et à son tour de la fibre laser en fonction du déplacement de la poulie de déflexion.

2. Urétéroscope selon la revendication 1, ladite sonde souple comprenant en outre :
un système de visualisation d'image conçu pour visualiser la fibre laser s'étendant depuis la pointe de déflexion et la calcul cible ; et
une source lumineuse conçue pour fournir de la lumière pour faciliter la visualisation de la fibre laser et du calcul cible par l'intermédiaire du système de visualisation d'image.

3. Urétéroscope selon la revendication 1, ledit module de traitement comprenant en outre :
un contrôleur comprenant :
une pluralité de modules d'interface d'entrée et de sortie conçus pour obtenir les signaux de réduction en poudre numérique provenant de la pluralité de commutateurs de sélection ; et
un module de prise de décision conçu pour déterminer le type de réduction en poudre du calcul cible, le type de réduction en poudre comprenant l'un d'une réduction en poudre fine du calcul cible et d'une réduction en poudre grossière du calcul cible.

4. Urétéroscope selon la revendication 1 ou 3, ladite poulie de déflexion couplée au moteur étant conçue pour dévier la pointe de déflexion en commandant la pluralité de câbles de commande dans un mouvement oscillant continu, ce qui permet de dévier la fibre laser dans le mouvement oscillant continu.

5. Urétéroscope selon la revendication 1 ou 3, ledit premier câble de commande étant dirigé à travers une première gaine, la première gaine étant contenue dans un premier support de paroi statique pour permettre au premier câble de commande de se déplacer sans frottements.

6. Urétéroscope selon la revendication 5, ledit second câble de commande étant dirigé à travers une seconde gaine, la seconde gaine étant contenue dans un second support de paroi statique pour permettre au second câble de commande de se déplacer sans frottements.

7. Urétéroscope selon la revendication 6, ledit premier câble de commande étant conçu pour se tendre en réponse au levier de déflexion qui est dévié vers une position à gauche et ledit second câble de commande étant conçu pour se relâcher pour permettre à la pointe de déflexion de se courber vers la gauche selon l'importance du tour dans le levier de déflexion.

8. Urétéroscope selon la revendication 7, ledit second câble de commande étant conçu pour se tendre en réponse au levier de déflexion qui est dévié vers une position à droite et ledit premier câble de commande étant conçu pour se relâcher pour permettre à la pointe de déflexion de se courber vers la droite selon l'importance du tour dans le levier de déflexion.

9. Urétéroscope selon l'une quelconque des revendications précédentes, ladite position de travail étant une distance prédéfinie de la fibre laser depuis l'extrémité distale de la sonde souple et étant définie par des paramètres d'énergie laser et la composition du calcul cible.

10. Urétéroscope selon l'une quelconque des revendications précédentes, ladite fibre laser étant conçue pour être énergisée jusqu'à un certain niveau de puissance et un certain niveau de fréquence pour permettre la réduction en poudre du calcul cible.

11. Urétéroscope selon la revendication 3 ou l'une quelconque des revendications dépendantes de celle-ci, ladite direction de déflexion et ladite magnitude de déflexion étant effectuées par la programmation du contrôleur à un signal sinusoïdal.
